(19) Europäisches Patentamt
European Patent Office
Office européen des brevets

(11) Publication number: **0 293 880 B1**

(12) **EUROPEAN PATENT SPECIFICATION**

(45) Date of publication of patent specification: **26.08.92**

(51) Int. Cl.[5]: **C07C 275/28**, C07D 333/20, A61K 31/17, A61K 31/38

(21) Application number: **88108816.5**

(22) Date of filing: **01.06.88**

# (54) Antihyperlipidemic and antiatherosclerotic urea compounds.

(30) Priority: **02.06.87 US 57576**
**05.02.88 US 147037**

(43) Date of publication of application:
**07.12.88 Bulletin 88/49**

(45) Publication of the grant of the patent:
**26.08.92 Bulletin 92/35**

(84) Designated Contracting States:
**AT BE CH DE ES FR GB GR IT LI LU NL SE**

(56) References cited:
**EP-A- 0 049 538**
**DD-A- 154 216**
**US-A- 4 387 105**
**US-A- 4 623 662**

(73) Proprietor: **WARNER-LAMBERT COMPANY**
**201 Tabor Road**
**Morris Plains New Jersey 07950(US)**

(72) Inventor: **Trivedi, Bharat Kalidas**
**44833 Tillotson Drive**
**Canton Mitchigan 48187(US)**

(74) Representative: **Mansmann, Ivo**
**c/o Gödecke AG - Patentabteilung Postfach**
**569 Mooswaldallee 1-9**
**W-7800 Freiburg(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 293 880 B1

## Description

This invention relates to urea compounds having pharmacological activity, to pharmaceutical compositions which include these compounds, and to the use thereof for the preparation of these compositions. More particularly, this invention concerns certain substituted urea compounds which inhibit the enzyme acyl-coenzyme A: cholesterol acyltransferase (ACAT), pharmaceutical compositions containing these compounds, and a method of use for the preparation of pharmaceuticals for inhibiting intestinal absorption of cholesterol or for regulating cholesterol.

In recent years the role which elevated blood plasma levels of cholesterol plays in pathological conditions in man has received much attention. Deposits of cholesterol in the vascular system have been indicated as causative of a variety of pathological conditions including coronary heart disease.

Initially, studies of this problem were directed toward finding therapeutic agents which would be effective in lowering total serum cholesterol levels. It is now known that cholesterol is transported in the blood in the form of complex particles consisting of a core of cholesterol esters plus triglycerides and an exterior consisting primarily of phospholipids and a variety of types of protein which are recognized by specific receptors. For example, cholesterol is carried to the sites of deposit in blood vessels in the form of low density lipoprotein cholesterol (LDL cholesterol) and away from such sites of deposit by high density lipo-protein cholesterol (HDL cholesterol).

Following these discoveries, the search for therapeutic agents which control serum cholesterol turned to finding compounds which are more selective in their action; that is, agents which are effective in elevating the blood serum levels of HDL cholesterol and/or lowering the levels of LDL cholesterol. While such agents are effective in moderating the levels of serum cholesterol, they have little or no effect on controlling the initial absorption of dietary cholesterol in the body through the intestinal wall.

In intestinal mucosal cells, dietary cholesterol is absorbed as free cholesterol which must be esterified by the action of the enzyme acyl-CoA: cholesterol acyltransferase (ACAT) before it can be packaged into the chylomicrons which are then released into the blood stream. Thus, therapeutic agents which effectively inhibit the action of ACAT prevent the intestinal absorption of dietary cholesterol into the blood stream or the reabsorption of cholesterol which has been previously released into the intestine through the body's own regulatory action.

The present invention provides a class of compounds with ACAT inhibitory activity having the formula

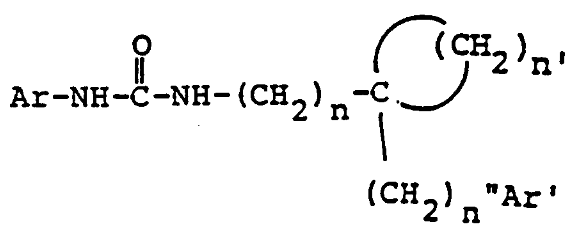

wherein Ar is unsubstituted
phenyl or
naphthyl, or
phenyl or naphthyl substituted with
alkyl of from one to six carbon atoms,
alkoxy of from one to six carbon atoms,
hydroxy,
fluorine,
chlorine,
bromine,
nitro,
trifluoromethyl, or
$-NR_1R_2$ wherein
$R_1$ and $R_2$ are independently hydrogen, or
alkyl of from one to six carbon atoms;
n is zero or is an integer of from one to three;
n' is an integer of from two to six; and

n" is zero, one, or two;
Ar' is selected from the group
phenyl,
naphthyl,
thienyl or
pyridinyl, which may be unsubstituted or substituted with
alkyl of from one to six carbon atoms,
alkoxy of from one to six carbon atoms,
hydroxy,
fluorine,
chlorine,
bromine,
nitro,
trifluoromethyl, or
$-NR_1R_2$ wherein
$R_1$ and $R_2$ are independently hydrogen or
alkyl of from one to six carbon atoms.

The compounds of the present invention form a class of substituted ureas having potent activity as inhibitors of the enzyme acyl CoA: cholesterol acyl-transferase (ACAT).

In the urea compounds of the present invention, the first nitrogen atom is monosubstituted by an aromatic ring system selected from phenyl or naphthyl. The phenyl ring is unsubstituted or, alternatively, is substituted with one, two, or three groups selected independently from alkyl of from one to six carbon atoms, alkoxy of from one to six carbon atoms, hydroxy, fluorine, chlorine, bromine, nitro, trifluoromethyl, or $-NR_1R_2$ in which $R_1$ and $R_2$ are independently hydrogen or alkyl of from one to six carbon atoms. Preferred compounds are those in which the aromatic ring system is phenyl or substituted phenyl.

In the urea compounds of this invention, the second nitrogen atom is substituted with an aryl-substituted cycloalkyl ring which may be attached directly to the nitrogen atom, or may separated from the nitrogen atom by a bridging group of up to three methylene (i.e.$-CH_2-$) groups. The cycloalkyl ring is cyclopropyl, cyclobutyl, cyclopentyl, cyclohexyl or cycloheptyl with cyclopentyl and cyclohexyl being preferred.

The cycolalkyl ring is further substituted, at the same atom of attachment to the nitrogen of the urea moiety or the same atom of attachment to the methylene bridge, by an aryl group. This aryl group is unsubstituted phenyl, naphthyl, thienyl, or pyridinyl or, alternatively, may be one of these aromatic rings optionally substituted by one, two, or three groups independently selected from alkyl of from one to six carbon atoms, alkoxy of from one to six carbon atoms, hydroxy, fluorine, chlorine, bromine, nitro, trifluoromethyl, or $-NR_1R_2$ in which $R_1$ and $R_2$ are independently hydrogen or alkyl of from one to six carbon atoms. Preferred compounds of this invention are those in which the aromatic ring system attached to the cycloalkyl ring is thienyl or unsubstituted phenyl.

Examples of compounds contemplated as falling within the scope of the invention are the following:

N-(2,6-Dimethylphenyl)-N'-(1-phenylcyclopentyl)urea
N-(2,6-Diethylphenyl)-N'-(1-phenylcyclobutyl)urea.
N-(2,6-Diethylphenyl)-N'-(1-phenylcyclopentyl)urea.
N-(2,6-Diethylphenyl)-N'-[(1-phenylcyclopropyl)methyl]urea.
N-(1-Phenylcyclopentyl)-N'-(2,4,6-trimethoxyphenyl)urea.
N-(2,6-Dimethylphenyl)-N'-[1-(2-thienyl)cyclohexyl]urea.
N-(2,6-Diethylphenyl)-N'-[1-(2-thienyl)cyclohexyl]urea.
N-[2,6-bis(1-Methylethyl)]-N'-[1-(2-thienyl)cyclohexyl]urea.
N-(2,6-Diethylphenyl)-N'-[(1-phenylcyclohexyl)methyl]urea.
N-(2,6-Dimethylphenyl)-N'-[(1-phenylcyclopentyl)methyl]urea.
N-(2,6-Dimethylphenyl)-N'-[(1-phenylcyclohexyl)methyl]urea.
N-(2,6-Diethylphenyl)-N'-[(1-phenylcyclopentyl)methyl]urea.
N-[2,6-bis(1-Methylethyl)phenyl]-N'-[(1-phenylcyclopentyl)methyl]urea.
N-[2,6-bis(1-Methylethyl)phenyl]-N'-[(1-phenylcyclohexyl)methyl]urea.
N-[2-Methyl-6-(1-methylethyl)phenyl]-N'-[1-(2-thienyl)cyclohexyl]urea.
N-[2-(1,1-Dimethylethyl)-6-methylphenyl]-N'-[1-(2-thienyl)cyclohexyl]urea.
N-[2-Methyl-6-(1-methylethyl)phenyl-N'-[(1-phenylcyclohexyl)methyl]urea.
N-[2-(1,1-Dimethylethyl)-6-methylphenyl]-N'-[(1-phenylcyclohexyl)methyl]urea.
N-[2-Ethyl-6-(1-methylethyl)phenyl]-N'-[(1-phenylcyclohexyl)methyl]urea.
N-[2-Methyl-6-(1-methylethyl)phenyl]-N'-[(1-phenylcyclopentyl)methyl]urea.

N-[2-(1,1-Dimethylethyl)-6-methylphenyl]-N'-[(1-phenylcyclopentyl)methyl]urea.
N-[2-Ethyl-6-(1-methylethyl)phenyl]-N'-[(1-phenylcyclopentyl)methyl]urea.
N-(2,4-Difluorophenyl)-N'-[1-(2-thienyl)-cyclohexyl]urea.
N-(2,4-Difluorophenyl)-N'-[(1-phenylcyclopentyl)methyl]urea.
N-(2,4-Difluorophenyl)-N'-[(1-phenylcyclohexyl)methyl]urea.
N-(2,6-Dibromo-4-fluorophenyl)-N'-[(1-phenylcyclohexyl)methyl]urea.

By the term "alkyl" as used throughout this specification and the appended claims is meant a branched or unbranched hydrocarbon grouping derived from a saturated hydrocarbon of from one to six carbon atoms by removal of a single hydrogen atom. Examples of alkyl groups contemplated as falling within the scope of this invention include methyl, ethyl, propyl, 1-methylethyl, butyl, 1-methylpropyl, 2-methylpropyl, and 1,1-dimethylethyl.

By the term "alkoxy" is meant an alkyl group, as defined above, attached to the parent molecular moiety through an oxygen atom.

In those instances where the compounds of the present invention bear a basic nitrogen atom as, for example, when Ar or Ar' is substituted by amino, alkylamino, or dialkylamino, or when Ar' is pyridinyl, the compounds are capable of forming acid addition salts. These acid addition salts are also contemplated as falling within the scope of this invention.

While the acid addition salts may vary from the free base form of the compounds in certain properties such as melting point and solubility, they are considered equivalent to the free base forms for the purposes of this invention.

The acid addition salts may be generated from the free base forms of the compounds by reaction of the latter with one equivalent of a suitable non-toxic, pharmaceutically acceptable acid, followed by evaporation of the solvent employed for the reaction and recrystallization of the salt, if required. The free base may be recovered from the acid addition salt by reaction of the salt with a water solution of the salt with a suitable base such as sodium carbonate, sodium bicarbonate, potassium carbonate, sodium hydroxide, and the like.

Suitable acids for forming acid addition salts of the compounds of this invention include, but are not necessarily limited to acetic, benzoic, benzenesulfonic, tartaric, hydrobromic, hydrochloric, citric, fumaric, gluconic, glucuronic, glutamic, lactic, malic, maleic, methanesulfonic, pamoic, salicylic, stearic, succinic, sulfuric, and tartaric acids. The class of acids suitable for the formation of non-toxic, pharmaceutically acceptable salts is well known to practitioners of the pharmaceutical formulation arts. (See, for example, Stephen N. Berge, et al. J. Pharm. Sciences, 66:1-19 (1977).

Further, the compounds of this invention may exist in unsolvated as well as solvated forms with pharmaceutically acceptable solvents such as water, ethanol and the like. In general, the solvated forms are considered equivalent to the unsolvated forms for the purposes of this invention.

The compounds of the present invention are prepared by the general method outline in Reaction Scheme 1. The appropriately substituted isocyanate, 2 are reacted with the desired amine, 3, to obtain the substituted urea compounds of the present invention.

The reaction is generally carried out in a polar aprotic organic solvent such as ethyl acetate, at any temperature between room temperature and the boiling point of the solvent, with room temperature being preferred.

The reaction is allowed to proceed until analysis of the mixture by a means such as chromatography indicates that the reaction is substantially complete. Reaction times may vary between about two hours to about 24 hours, depending upon the particular reagents and reaction temperature employed. The starting isocyanate compounds are known or commercially available or, if not previously known, are prepared by methods well known in the art from the corresponding amine compounds.

The amine compounds, 3, are prepared by the general method detailed in J. Org. Chem., 36(9): 1308 (1971) and depicted schematically in Reaction Scheme 2. Referring to Reaction Scheme 2, phenylacetonitrile or the substituted phenylacetonitrile 5, is reacted with the desired alpha-omega dibromoalkalne, 6, in the presence of base to produce the cycloalkyl nitrile, 7. This compound

Reaction Scheme I

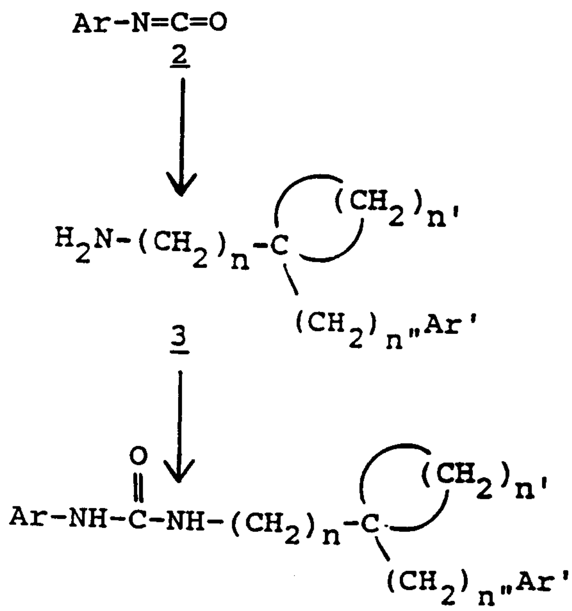

Reaction Scheme 2

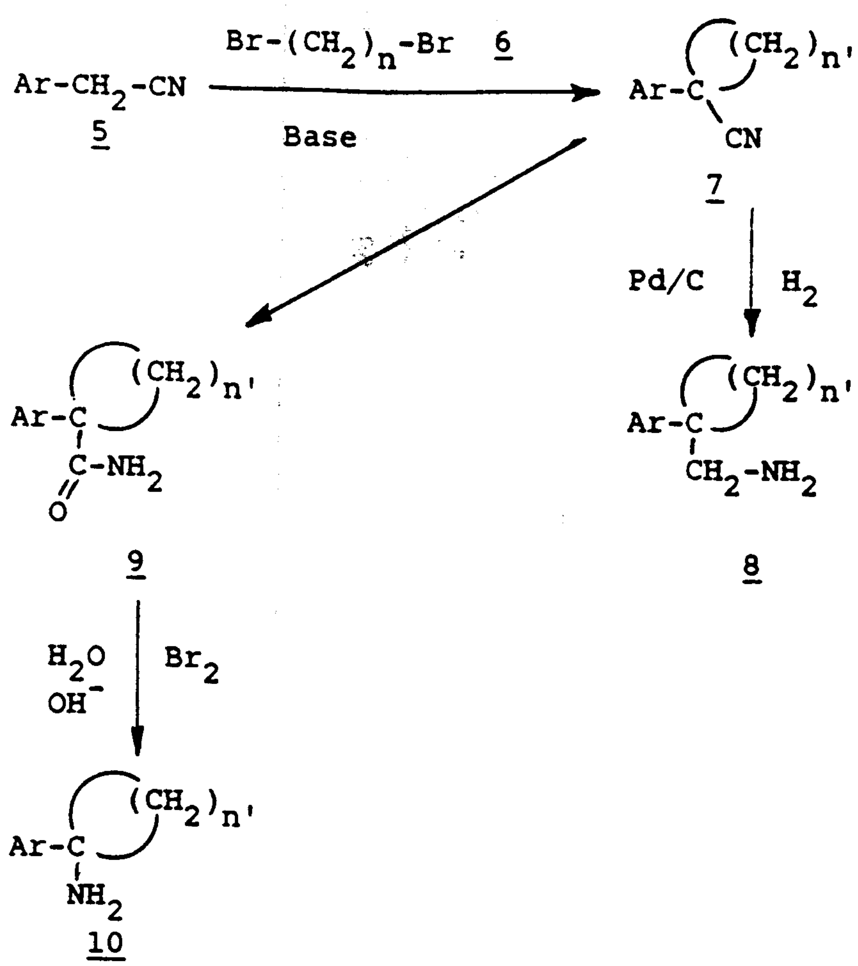


5 7 8 9 10

is catalytically reduced by the action of hydrogen over a noble metal catalyst to produce the aryl-(aminomethyl)cycloalkane compound, 8. Acid hydrolysis of compound 6, produces the corresponding aryl-(cycloalkyl) carboxamide, 9, which is then subjected to the Hofmann degradation reaction (Ber., 14: 2725 (1881)) to produce the aryl(cycloalkyl)amine 10.

As shown by the data presented below in Table 1, the compounds of the present invention are potent inhibitors of the enzyme acyl-CoA:cholesterol acyl-transferase (ACAT), and are thus effective in inhibiting the esterification and transport of cholesterol across the intestinal cell wall. The compounds of the present invention are thus useful in pharmaceutical formulations for the inhibition of intestinal absorption of dietary cholesterol, the reabsorption of cholesterol released into the intestine by normal body action, or the modulation of cholesterol.

In Vitro Tests

The ability of representative compounds of the present invention to inhibit ACAT was measured using an in vitro test more fully described in Field, F. J. and Salone, R. G., Biochemica et Biophysica 712: 557-570 (1982). The test assesses the ability of a test compound to inhibit the acylation of cholesterol by oleic acid by measuring the amount of radio-labeled cholesterol oleate formed from radio-labeled oleic acid in a tissue preparation containing rabbit intestinal microsomes.

The data appear in Table 1 where they are expressed as $IC_{50}$ values; i.e. the concentration of test compound required to inhibit 50% expression of the enzyme.

Table 1

| Compound of Example | $IC_{50}$ (Micromolar) |
|---|---|
| 1 | 0.08 |
| 2 | 0.23 |
| 3 | 0.12 |
| 4 | 0.088 |
| 5 | 0.70 |
| 6 | 0.13 |
| 7 | 0.048 |
| 8 | 0.043 |
| 9 | 0.051 |
| 10 | 0.154 |
| 11 | 0.081 |
| 12 | 0.015 |
| 13 | 0.017 |
| 14 | 0.021 |
| 15 | 0.23 |
| 16 | 0.256 |
| 17 | 0.058 |
| 18 | 0.054 |
| 19 | 0.020 |
| 20 | 0.016 |
| 21 | 0.018 |
| 22 | 0.025 |
| 23 | 0.37 |
| 24 | 1.28 |
| 25 | 0.79 |
| 26 | 0.039 |

In Vivo Tests

In one in vivo screen, designated PCC, male Sprague-Dawley rats (approximately 200 g body weight) are randomly divided into groups and provided ad libidum a regular chow diet (Purina No. 5002, Ralston Purina Co., 711 West Fuesser Road, Mascoutah, Illinois, 62224, USA), supplemented with 5.5% peanut oil, 1.5% cholesterol, and 0.3%-0.5% cholic acid, together with 0.05% of the test drug which is admixed into the diet. After one week the animals are etherized and a blood sample is taken from the heart and mixed with 0.14% ethylenediamine tetraacetic acid (EDTA) to measure the total cholesterol. The results of this trial for representative compounds of the present invention appear in Table 2.

Table 2

| Compound of Example | Total Blood Cholesterol (mg/dl) | % Change |
|---|---|---|
| 13 | 61 (Control = 224) | -73 |
| 8 | 60 (Control = 181) | -69 |

In therapeutic use as agents for the inhibition of intestinal absorption of cholesterol, or as hypolipidemic or hypocholesterolemic agents, the compounds utilized in the pharmaceutical method of this invention are administered to the patient at dosage levels of from 250 to 1000 mg per day. For a normal human adult of approximately 70 kg of body weight, this translates into a dosage of from 5 to 20 mg/kg of body weight per day. The specific dosages employed, however, may be varied depending upon the requirements of the patient, the severity of the condition being treated, and the activity of the compound being employed. The

determination of optimum dosages for a particular situation is within the skill of the art.

For preparing pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

Powders and tablets preferably contain between about 5 to about 70% by weight of the active ingredient. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner, cachets are also included.

Tablets, powders, cachets, and capsules can be used as solid dosage forms suitable for oral administration.

Liquid form preparations include solutions suitable for oral administration, or suspensions and emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

The following preparative examples are provided to enable one skilled in the art to practice the invention, and are illustrative thereof. They are not to be read as limiting the scope of the invention as it is defined by the appended claims.

Example 1

Preparation of N-(2,6-dimethylphenyl)-N'-(1-phenylcyclopentyl)urea

To a solution of 1-phenylcyclopentyl amine (1.0 g, 0.006 mole) in 30 ml of ethylacetate, 2,6-dimethylphenyl isocyanate (0.91 g, 0.006 mole) is added and the reaction mixture is stirred at room temperature for 20 hours Volatiles are removed under reduced pressure and the residue is crystallized from ethylacetate-hexane yielding 1.65 g of N-(2,6-dimethylphenyl -N'-(1-phenylcyclopentyl) urea having a melting point of 227-229° C

| Analysis calculated for $C_{20}H_{24}N_2O$ | | | |
|---|---|---|---|
| | C = 77.88, | H = 7.84; | N = 9.08 |
| Found | C = 77.72; | H = 7.83; | N = 9.19 |

Example 2

Preparation of N-(2,6-diethylphenyl)-N'-(1-phenylcyclobutyl)urea

To a solution of 1-phenylcyclobutyl amine (1.0 g, 0.0057 mole) in 30 ml of ethylacetate, 2,6-diethylphenylisocyanate (0.84 g, 0.0057 mole) is added and the reaction mixture is stirred at room temperature for 20 hours. Precipitated solid is filtered, washed with ethylacetate and dried yielding 1.46 g of

N-(2, 6-diethylphenyl)-N'-(1-phenylcyclobutyl)urea having a melting point of 227-230 ° C.

| Analysis calculated for $C_{21}H_{26}N_{20}$ | | | |
|---|---|---|---|
| | C = 78.22; | H = 8.12; | N = 8.68 |
| Found | C = 78.43; | H = 8.32; | N = 8.89 |

Example 3

Preparation of N-(2,6-diethylphenyl)-N'-(1-phenylcyclopentyl)urea

The title compound is prepared according to the procedure described for the Example 2. Melting point 215-218 ° C.

| Analysis calculated for $C_{22}H_{28}N_2O$ | | | |
|---|---|---|---|
| | C = 78.53; | H = 8.38; | N = 8.32 |
| Found | C = 78.35, | H = 8.34; | N = 8.19 |

Example 4

Preparation of N-(2,6-diethylphenyl)-N'-[(1-phenylcyclopropyl)methyl]urea

The title compound is prepared according to Example 2. Melting point 166-168 ° C

| Analysis calculated for $C_{21}H_{26}N_2O$ | | | |
|---|---|---|---|
| | C = 78.22; | H = 8.12; | N = 8.68 |
| Found | C = 78.13; | H = 8.28; | N = 8.63 |

Example 5

Preparation of N-(1-phenylcyclopentyl)-N'-(2,4,6-trimethoxyphenyl)urea

The title compound is prepared according to Example 2. Melting point 193-195 ° C

| Analysis calculated for $C_{21}H_{26}N_2O_4$ | | | |
|---|---|---|---|
| | C = 68.09; | H = 7.07; | N = 7.56 |
| Found | C = 67.75; | H = 6.85; | N = 7.34 |

Example 6

Preparation of N-(2,6-dimethylphenyl)-N'-[1-(2-thienyl)cyclohexyl]urea

The title compound is prepared according to Example 2. Melting point 220-222 ° C.

| Analysis calculated for $C_{19}H_{24}N_2OS$ | | | | |
|---|---|---|---|---|
| | C = 69.47; | H = 7.36; | N = 8.52, | S = 9.76 |
| Found | C = 69.43; | H = 7.24; | N = 8.69; | S = 9.87 |

Example 7

Preparation of N-(2,6-diethylphenyl)-N'-[1-(2-thienyl)cyclohexyl]urea

The title compound is prepared according to Example 2. Melting Point 208-210 ° C

| Analysis calculated for $C_{21}H_{28}N_2OS$ | | | | |
|---|---|---|---|---|
| | C = 70.75; | H = 7.91; | N = 7.85; | S = 8.99 |
| Found | C = 71.02; | H = 8.08; | N = 7.93; | S = 9.08 |

Example 8

Preparation of N-[2,6-bis(1-methylethyl)]-N'-[1-(2-thienyl)cyclohexyl]urea

The title compound is prepared according to Example 2. Melting point 170-171 ° C.

| Analysis calculated for $C_{23}H_{32}N_2OS$ | | | | |
|---|---|---|---|---|
| | C = 71.83; | H = 8.38; | N = 7.28; | S = 8.33 |
| Found | C = 72.18; | H = 8.48; | N = 7.37, | S = 8.64 |

Example 9

Preparation of N-(2,6-diethylphenyl)-N'-[(1-phenylcyclohexyl)methyl]urea

The title compound is prepared according to Example 2. Melting point 185-186 ° C.

| Analysis calculated for $C_{24}H_{32}N_2O$ | | | |
|---|---|---|---|
| | C = 79.08; | H = 8.84; | N = 7.68 |
| Found | C = 79.42; | H = 8.95; | N = 7.70 |

Example 10

Preparation of N-(2,6-dimethylphenyl)-N'-[(1-phenylcyclopentyl)methyl]urea

The title compound is prepared according to Example 2. Melting point 201-202 ° C.

| Analysis calculated for $C_{21}H_{26}N_2O$ | | | |
|---|---|---|---|
| | C = 78.22; | H = 8.12; | N = 8.68 |
| Found | C = 77.92; | H = 8.05; | N = 8.64 |

Example 11

Preparation of N-(2,6-dimethylphenyl)-N'-[(1-phenylcyclohexyl)methyl]urea

The title compound is prepared according to Example 2. Melting point 207-208 ° C.

| Analysis calculated for $C_{22}H_{28}N_2O$ | | | |
|---|---|---|---|
| | C = 78.53; | H = 8.38; | N = 8.32 |
| Found | C = 78.59; | H = 8.37; | N = 8.46 |

Example 12

Preparation of N-(2,6-diethylphenyl)-N'-[(1-phenylcyclopentyl)methyl]urea

The title compound is prepared according to Example 2. Melting point 165-167°C.

| Analysis calculated for $C_{23}H_{30}N_2O$ | | | |
|---|---|---|---|
| | C = 78.62; | H = 8.62; | N = 7.99 |
| Found | C = 78.54; | H = 8.48; | N = 7.93 |

Example 13

Preparation of N-[2,6-bis(1-methylethyl)phenyl]-N'-[(1-phenylcyclopentyl)methyl]urea

The title compound is prepared according to Example 2. Melting point 181-183°C.

| Analysis calculated for $C_{25}H_{34}N_2O$ | | | |
|---|---|---|---|
| | C = 79.32; | H = 9.05; | N = 7.39 |
| Found | C = 79.01; | H = 8.97; | N = 7.21 |

Example 14

Preparation of N-[2,6-bis(1-methylethyl)phenyl-N'-[(1-phenylcyclohexyl)methyl]urea

The title compound is prepared according to Example 2. Melting point 168-169°C.

| Analysis calculated for $C_{26}H_{36}N_2O$ | | | |
|---|---|---|---|
| | C = 79.55; | H = 9.24; | N = 7.13 |
| Found | C = 79.31; | H = 8.99; | N = 7.06 |

Example 15

Preparation of N-[2-methyl-6-(1-methylethyl)-phenyl]-N'-[1-(2-thienyl)cyclohexyl]urea

The title compound is prepared according to Example 2. Melting point 230-232°C.

| Analysis calculated for $C_{21}H_{28}N_2OS$ | | | | |
|---|---|---|---|---|
| | C = 70.74; | H = 7.91; | N = 7.85; | S = 8.99 |
| Found | C = 70.53; | H = 7.88; | N = 8.03; | S = 8.90 |

Example 16

Preparation of N-[2-(1,1-dimethylethyl)-6-methyl-phenyl]-N'-[1-(2-thienyl)cyclohexyl]urea

The title compound is prepared according to Example 2. Melting point 245-246 ° C.

| Analysis calculated for $C_{22}H_{30}N_2OS$ | | | | |
|---|---|---|---|---|
| | C = 71.31; | H = 8.16; | N = 7.55; | S = 8.65 |
| Found | C = 71.51; | H = 8.19; | N = 7.53; | S = 8.43 |

Example 17

Preparation of N-[2-methyl-6-(1-methylethyl)-phenyl]-N'-[(1-phenylcyclohexyl)methyl]urea

The title compound is prepared according to Example 2. Melting point 158-160 ° C.

| Analysis calculated for $C_{24}H_{32}N_2O$ | | | |
|---|---|---|---|
| | C = 79.08; | H = 8.84; | N = 7.68 |
| Found | C = 78.75; | H = 8.89; | N = 7.76 |

Example 18

Preparation of N-[2-(1,1-dimethylethyl)-6-methylphenyl]-N'-[(1-phenylcyclohexyl)methyl]-urea

The title compound is prepared according to Example 2. Melting point 196-197 ° C.

| Analysis calculated for $C_{25}H_{34}N_2O$ | | | |
|---|---|---|---|
| | C = 79.32; | H = 9.05; | N = 7.39 |
| Found | C = 79.11; | H = 9.27; | N = 7.36 |

Example 19

Preparation of N-[2-ethyl-6-(1-methylethyl)-phenyl]-N'-[(1-phenylcyclohexyl)methyl]urea

The title compound is prepared according to Example 2. Melting point 178-180 ° C.

| Analysis calculated for $C_{25}H_{34}N_2O$ | | | |
|---|---|---|---|
| | C = 79.32; | H = 9.05; | N = 7.39 |
| Found | C = 79.62; | H = 9.17; | N = 7.47 |

Example 20

Preparation of N-[2-methyl-6-(1-methylethyl)-phenyl]-N'-[(1-phenylcyclopentyl)methyl]urea

The title compound is prepared according to Example 2. Melting point 157-159 ° C.

| Analysis calculated for $C_{23}H_{30}N_2O$ | | | |
|---|---|---|---|
| | C = 78.81; | H = 8.62; | N = 7.99 |
| Found | C = 78.48; | H = 8.61; | N = 7.93 |

Example 21

Preparation of N-[2-(1,1-dimethylethyl)-6-methylphenyl]-N'-[(1-phenylcyclopentyl)methyl]-urea

The title compound is prepared according to Example 2. Melting point 196-197 °C.

| Analysis calculated for $C_{24}H_{32}N_2O$ | | | |
|---|---|---|---|
| | C = 79.08; | H = 8.84; | N = 7.68 |
| Found | C = 78.65; | H = 9.01; | N = 7.58 |

Example 22

Preparation of N-[2-ethyl-6-(1-methylethyl)-phenyl]-N'-[(1-phenylcyclopentyl)methyl]urea

The title compound is prepared according to Example 2. Melting point 152-154 °C.

| Analysis calculated for $C_{24}H_{32}N_2O$ | | | |
|---|---|---|---|
| | C = 79.08; | H = 8.84; | N = 7.68 |
| Found | C = 79.17; | H = 9.05; | N = 7.65 |

Example 23

Preparation of N-(2,4-difluorophenyl)-N'-[1-(2-thienyl)cyclohexyl]urea

The title compound is prepared according to Example 1. Melting point 175-177 °C.

| Analysis calculated for $C_{17}H_{18}N_2OSF_2$ | | | | |
|---|---|---|---|---|
| | C = 60.70; | H = 5.39; | N = 8.32; | S = 9.53 |
| Found | C = 60.80; | H = 5.50; | N = 8.31; | S = 9.62 |

Example 24

Preparation of N-(2,4-difluorophenyl)-N'-[(1-phenylcyclopentyl)methyl]urea

The title compound is prepared according to Example 1. Melting point 160-161 °C.

| Analysis calculated for $C_{19}H_{20}N_2OF_2$ | | | | |
|---|---|---|---|---|
| | C = 69.08; | H = 6.10; | N = 8.47; | F = 11.49 |
| Found | C = 68.94; | H = 6.09; | N = 8.32; | F = 11.55 |

Example 25

Preparation of N-(2,4-difluorophenyl)-N'-[(1-phenylcyclohexyl)methyl]urea

The title compound is prepared according to Example 1. Melting point 163-165 ° C.

| Analysis calculated for $C_{20}H_{22}N_2OF_2$ | | | | |
|---|---|---|---|---|
| | C = 69.75; | H = 6.43; | N = 8.13; | F = 11.03 |
| Found | C = 69.58; | H = 6.56; | N = 8.04; | F = 10.87 |

Example 26

Preparation of N-(2,6-dibromo-4-fluorophenyl)-N'-[(1-phenyl-cyclohexyl)methyl]urea

The title compound is prepared according to Example 2. Melting point 196-198 ° C.

| Analysis calculated for $C_{20}H_{21}N_2OBr_2F$ | | | | | |
|---|---|---|---|---|---|
| | C = 49.61; | H = 4.37; | N = 5.78; | Br = 33.0; | F = 3.92 |
| Found | C = 49.66; | H = 4.32; | N = 5.68; | Br = 32.72; | F = 3.80 |

## Claims

**1.** A compound having the formula

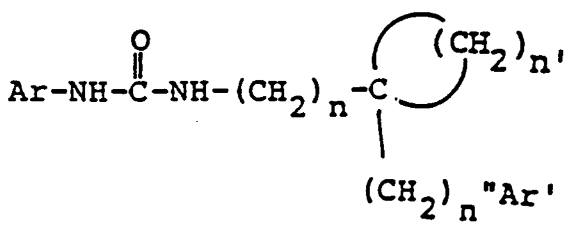

wherein Ar is unsubstituted
phenyl or
naphthyl, or
phenyl or naphthyl substituted with
alkyl of from one to six carbon atoms,
alkoxy of from one to six carbon atoms,
hydroxy,
fluorine,
chlorine,
bromine,
nitro,
trifluoromethyl, or
$-NR_1R_2$ wherein
$R_1$ and $R_2$ are independently hydrogen, or
alkyl of from one to six carbon atoms;
n is zero or is an integer of from one to three;
n' is an integer of from two to six; and
n" is zero, one, or two;
Ar' is selected from the group
phenyl,
naphthyl,
thienyl or

pyridinyl, which may be unsubstituted or
substituted with
alkyl of from one to six carbon atoms,
alkoxy of from one to six carbon atoms,
hydroxy,
fluorine,
chlorine,
bromine,
nitro,
trifluoromethyl, or
$-NR_1R_2$ wherein
$R_1$ and $R_2$ are independently hydrogen or
alkyl of from one to six carbon atoms;
or a pharmaceutically acceptable salt thereof.

**2.** A compound as defined by Claim 1 wherein Ar is phenyl or phenyl optionally substituted by
alkyl of from one to six carbon atoms,
alkoxy of from one to six carbon atoms,
hydroxy,
fluorine,
chlorine,
bromine,
nitro,
trifluoromethyl, or
$-NR_1R_2$ wherein
$R_1$ and $R_2$ are independently hydrogen, or
alkyl of from one to six carbon atoms.

**3.** A compound as defined by Claim 1 wherein Ar' is selected from unsubstituted
thienyl or
phenyl; or
phenyl or thienyl substituted with
alkyl of from one to six carbon atoms,
alkoxy of from one to six carbon atoms,
hydroxy,
fluorine,
chlorine,
bromine,
nitro,
trifluoromethyl, or
$-NR_1R_2$ wherein
$R_1$ and $R_2$ are independently hydrogen or
alkyl of from one to six carbon atoms.

**4.** A compound as defined by Claims 1 to 3 selected from the group N-(2,6-dimethylphenyl)-N-(1-phenylcyclopentyl)urea;
N-(2,6-diethylphenyl)-N'-(1-phenylcyclobutyl)urea;
N-(2,6-diethylphenyl)-N'-(1-phenylcyclopentyl)urea;
N-(2,6-diethylphenyl)-N'-[(1-phenylcyclopropyl)methyl]urea;
N-(1-phenylcyclopentyl)-N'-(2,4,6-trimethoxyphenyl)urea;
N-(2,6-dimethylphenyl)-N'-[1-(2-thienyl)cyclohexyl]urea;
N-(2,6-diethylphenyl)-N'-[1-(2-thienyl)cyclohexyl]urea;
N-[2,6-bis(1-methylethyl)]-N'-[1-(2-thienyl)cyclohexyl]urea;
N-(2,6-diethylphenyl)-N'-[(1-phenylcyclohexyl)methyl]urea;
N-(2,6-dimethylphenyl)-N'-[(1-phenylcyclopentyl)methyl]urea;
N-(2,6-dimethylphenyl)-N'-[(1-phenylcyclohexyl)methyl]urea;
N-(2,6-diethylphenyl)-N'-[(1-phenylcyclopentyl)methyl]urea;
N-[2,6-bis(1-methylethyl)phenyl]-N'-[(1-phenylcyclopentyl)methyl]urea

N-[2,6-bis(1-methylethyl)phenyl]-N'-[(1-phenylcyclohexyl)methyl]urea;
N-[2-methyl-6-(1-methylethyl)phenyl]-N'-[1-(2-thienyl)cyclohexyl]urea;
N-[2-(1,1-dimethylethyl)-6-methylphenyl]-N'-[1-(2-thienyl)cyclohexyl]urea;
N-[2-methyl-6-(1-methylethyl)phenyl]-N'-[(1-phenylcyclohexyl)methyl]urea;
N-[2-(1,1-dimethylethyl)-6-methylphenyl]-N'-[(1-phenylcyclohexyl)methyl]urea;
N-[2-ethyl-6-(1-methylethyl)phenyl]-N'-[(1-phenylcyclohexyl)methyl]urea;
N-[2-methyl-6-(1-methylethyl)phenyl]-N'-[(1-phenylcyclopentyl)methyl]urea;
N-[2-(1,1-dimethylethyl)-6-methylphenyl]-N'-[(1-phenylcyclopentyl)methyl]urea;
N-[2-ethyl-6-(1-methylethyl)phenyl]-N'-[(1-phenylcyclopentyl)methyl]urea;
N-(2,4-difluorophenyl)-N'-[1-(2-thienyl)cyclohexyl]urea;
N-(2,4-difluorophenyl)-N'-((1-phenylcyclopentyl)methyl]urea;
N-(2,4-difluorophenyl)-N'-[(1-phenylcyclohexyl)methyl]urea; or
N-(2,6-dibromo-4-fluorophenyl)-N'-[(1-phenylcyclohexyl)methyl]urea.

**5.** A pharmaceutical composition for regulating cholesterol comprising an ACAT-inhibitory effective amount of a compound as defined by Claims 1 to 4 in combination with a pharmaceutically acceptable carrier.

**6.** use of a compound according to claims 1 to 4 for the preparation of pharmaceuticals for the inhibition of intestinal absorption of cholesterol or for the regulation of cholesterol.

**7.** A process for the preparation of pharmaceuticals by formulation of an active compound as defined in claims 1 to 4 with an inert pharmaceutically acceptable carrier.

**8.** A method of preparing a compound as defined in claims 1 to 4 comprising the steps of
a) reacting a compound of the formula

Ar-N = C = O

wherein Ar is as defined above with a compound having the formula

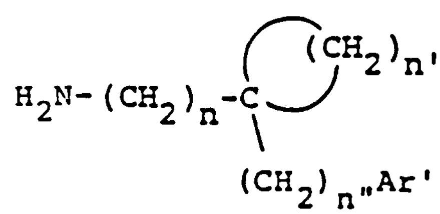

wherein n, n', n", and Ar' are as defined above in a polar aprotic organic solvent at ambient temperature;
b) thereafter isolating the product of said reaction step; and
c) if desired, converting said isolated product to a pharmaceutically acceptable salt.

**Patentansprüche**

**1.** Verbindungen der Formel

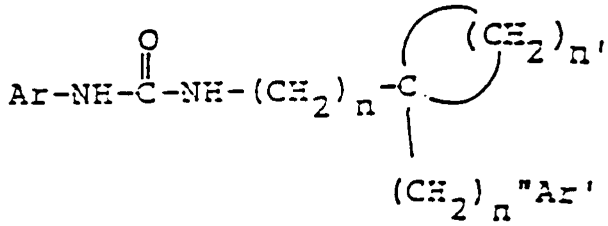

worin

Ar unsubstituiertes Phenyl oder Naphthyl oder durch Alkyl mit 1 bis 6 Kohlenstoffatomen, Alkoxy mit 1 bis 6 Kohlenstoffatomen, Hydroxy, Fluor, Chlor, Brom, Nitro, Trifluormethyl oder $-NR_1R_2$, worin $R_1$ und $R_2$ unabhängig voneinander Wasserstoff oder Alkyl mit 1 bis 6 Kohlenstoffatomen bedeuten, substituiertes Phenyl oder Naphthyl;

n Null oder eine ganze Zahl von 1 bis 3;

n' Null oder eine ganze Zahl von 2 bis 6;

n" Null, 1 oder 2; und

Ar' eine aus der aus unsubstituiertem oder durch Alkyl mit 1 bis 6 Kohlenstoffatomen, Alkoxy mit 1 bis 6 Kohlenstoffatomen, Hydroxy, Fluor, Chlor, Brom, Nitro, Trifluormethyl oder $-NR_1R_2$, wobei $R_1$ und $R_2$ unabhängig voneinander Wasserstoff oder Alkyl mit 1 bis 6 Kohlenstoffatomen bedeuten, substituiertem Phenyl, Naphthyl, Thienyl oder Pyridinyl bestehenden Gruppe ausgewählte Gruppe

bedeuten, oder deren pharmazeutisch annehmbaren Salze.

**2.** Verbindung nach Anspruch 1, worin

Ar gegebenenfalls durch Alkyl mit 1 bis 6 Kohlenstoffatomen, Alkoxy mit 1 bis 6 Kohlenstoffatomen, Hydroxy, Fluor, Chlor, Brom, Nitro, Trifluormethyl oder $-NR_1R_2$, worin $R_1$ und $R_2$ unabhängig voneinander Wasserstoff oder Alkyl mit 1 bis 6 Kohlenstoffatomen bedeuten, substituiertes Phenyl

bedeutet.

**3.** Verbindung nach Anspruch 1, worin

Ar' eine aus der aus unsubstituiertem Thienyl oder Phenyl oder durch Alkyl mit 1 bis 6 Kohlenstoffatomen, Alkoxy mit 1 bis 6 Kohlenstoffatomen, Hydroxy, Fluor, Chlor, Brom, Nitro, Trifluormethyl oder $-NR_1R_2$, worin $R_1$ und $R_2$ unabhängig voneinander Wasserstoff oder Alkyl mit 1 bis 6 Kohlenstoffatomen bedeuten, substituiertem Phenyl oder Thienyl bestehenden Gruppe ausgewählte Gruppe

bedeutet.

**4.** Verbindung, wie in den Ansprüchen 1 bis 3 definiert, ausgewählt aus der aus
N-(2,6-Dimethylphenyl)-N'-(1-phenylcyclopentyl)harnstoff;
N-(2,6-Diethylphenyl)-N'-(1-phenylcyclobuty)harnstoff;
N-(2,6-Diethylphenyl)-N'-(1-phenylcyclopentyl)harnstoff;
N-(2,6-Diethylphenyl)-N'-[(1-phenylcyclopropyl)methyl]-harnstoff;
N-(1-Phenylcyclopentyl)-N'-(2,4,6-trimethoxyphenyl)harnstoff;
N-(2,6-Dimethylphenyl)-N'-[1-(2-thienyl)cyclohexyl]harnstoff;
N-(2,6-Diethylphenyl)-N'-(1-(2-thienyl)cyclohexyl]harnstoff;
N-[2,6-Bis(1-methylethyl)]-N'-[1-(2-thienyl)cyclohexyl]harnstoff;
N-(2,6-Diethylphenyl)-N'-[(1-phenylcyclohexyl)methyl]harnstoff;
N-(2,6-Dimethylphenyl)-N'-[(1-phenylcyclopentyl)methyl]harnstoff;
N-(2,6-Dimethylphenyl)-N'-[(1-1-phenylcyclohexyl)methyl]harnstoff;
N-(2,6-Diethylphenyl)-N'-[(1-phenylcyclopentyl)methyl]harnstoff;
N-[2,6-Bis(1-methylethyl)phenyl]-N'-[(1-phenylcyclopentyl)methyl]harnstoff;
N-[2,6-Bis(1-methylethyl)phenyl]-N'-[(1-phenylcyclohexyl)methyl]harnstoff;
N-[2-Methyl-6-(1-methylethyl)phenyl]-N'-[1-(2-thienyl)cyclohexyl]harnstoff;
N-[2-(1,1-Dimethylethyl)-6-methylphenyl]-N'-[1-(2-thienyl)cyclohexyl)harnstoff;
N-[2-Methyl-6-(1-methylethyl)phenyl]-N'-[(1-phenylcyclohexyl)methyl]harnstoff;
N-[2-1,1-Dimethylethyl)-6-methylphenyl]-N'-[(1-phenylcyclohexyl)methyl]harnstoff;
N-[2-Ethyl-6-(1-methylethyl)phenyl]-N'-[(1-phenylcyclohexyl)methyl]harnstoff;
N-[2-Methyl-6-(1-methylethyl)phenyl]-N'-[(1-phenylcyclopentyl)methyl]harnstoff;
N-[2-(1,1-Dimethylethyl)-6-methylphenyl]-N'-[(1-phenylcyclopentyl)methyl]harnstoff;
N-[2-Ethyl-6-(1-methylethyl)phenyl]-N'-[(1-phenylcyclopentyl)methyl]harnstoff;
N-(2,4-Difluorphenyl)-N'-[1-(2-thienyl)cyclohexyl]harnstoff;
N-(2,4-Difluorphenyl)-N'-[(1-phenylcyclopentyl)methyl]harnstoff;
N-(2,4-Difluorphenyl)-N'-[(1-phenylcyclohexyl)methyl]harnstoff; und
N-(2,6-Dibrom-4-fluorphenyl)-N'-[(1-phenylcyclohexyl)methyl]harnstoff
bestehenden Gruppe.

5. Pharmazeutische Zubereitung zur Cholesterinregulierung mit einem Gehalt an einer Verbindung, wie in den Ansprüchen 1 bis 4 definiert, in einer Menge, welche eine wirksame ACAT-Hemmung bewirkt, in Kombination mit einem pharmazeutisch annehmbaren Trägerstoff.

6. Verwendung einer Verbindung nach einem der Ansprüche 1 bis 4 zur Herstellung von Arzneimitteln, welche die intestinale Absorption von Cholesterin hemmen oder die Regulierung von Cholesterin bewirken.

7. Verfahren zur Herstellung von Arzneimitteln durch Formulieren einer aktiven Komponente, wie in den Ansprüchen 1 bis 4 definiert, mit einem inerten pharmazeutisch annehmbaren Trägerstoff.

8. Verfahren zur Herstellung einer Verbindung, wie in den Ansprüchen 1 bis 4 definiert, welches die folgenden Verfahrensschritte aufweist:

a) Umsetzung einer Verbindung der Formel

Ar-N = C = O ,

worin Ar wie oben definiert ist, mit einer Verbindung der Formel

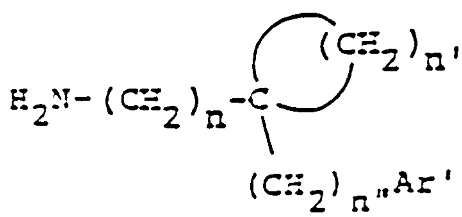

worin n, n', n" und Ar' die oben angegebenen Bedeutungen haben, in einem polaren aprotischen Lösungsmittel bei Raumtemperatur;

b) danach Isolierung des Produktes aus dieser Umsetzung; und

c) Ueberführen des Isolierten Produktes, sofern erwünscht, in ein pharmazeutisch annehmbares Salz.

**Revendications**

1. Un composé ayant la formule:

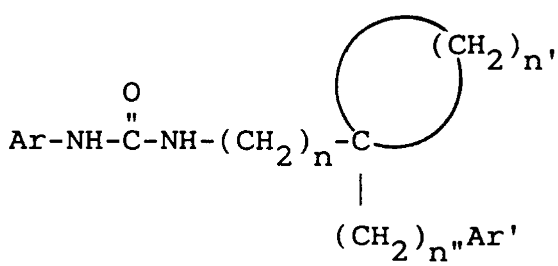

où:

Ar est un radical phényle ou naphtyle non substitué, ou phényle ou naphtyle substitué par un ou plusieurs radicaux alkyles ayant 1 à 6 atomes de carbone, alcoxy ayant 1 à 6 atomes de carbone, hydroxy, fluoro, chloro, bromo, nitro, trifluorométhyle, ou $-NR_1R_2$ où $R_1$ et $R_2$, indépendamment l'un de l'autre, sont de l'hydrogène ou un radical alkyle ayant 1 à 6 atomes de carbone;

n vaut 0 ou est un entier de 1 à 3;

n' est un entier de 2 à 6; et

n" vaut 0, 1 ou 2;

Ar' est choisi dans l'ensemble comprenant les radicaux phényle, naphtyle, thiényle ou pyridinyle, qui peuvent être non-substitués ou encore substitués par un ou plusieurs radicaux alkyles

ayant 1 à 6 atomes de carbone, alcoxy ayant 1 à 6 atomes de carbone, hydroxy, fluoro, chloro, bromo, nitro, trifluorométhyle, ou $-NR_1R_2$
où $R_1$ et $R_2$, indépendamment l'un de l'autre, sont de l'hydrogène ou un radical alkyle ayant 1 à 6 atomes de carbone;
ou l'un de ses sels pharmaceutiquement acceptable.

**2.** Un composé selon la revendication 1, dans lequel:
Ar est un radical phényle, ou phényle éventuellement substitué par un radical alkyle ayant 1 à 6 atomes de carbone, alcoxy ayant 1 à 6 atomes de carbone, hydroxy, fluoro, chloro, bromo, nitro, trifluorométhyle, ou $-NR_1R_2$
où $R_1$ et $R_2$, indépendamment l'un de l'autre, sont de l'hydrogène ou un radical alkyle ayant 1 à 6 atomes de carbone.

**3.** Un composé selon la revendication 1, dans lequel:
Ar' est choisi parmi les radicaux thiényle ou phényle non substitués; ou les radicaux phényle ou thiényle substitués par un ou plusieurs radicaux alkyles ayant 1 à 6 atomes de carbone, alcoxy ayant 1 à 6 atomes de carbone, hydroxy, fluoro, chloro, bromo, nitro, trifluorométhyle, ou $-NR_1R_2$
où $R_1$ et $R_2$, indépendamment l'un de l'autre, sont de l'hydrogène ou un radical alkyle ayant 1 à 6 atomes de carbone.

**4.** Un composé selon les revendications 1 à 3, choisi dans le groupe comprenant les composés suivants:
. N-(2,6-diméthylphényl)-N'-(1-phénylcyclopentyl)urée,
. N-(2,6-diéthylphényl)-N'-(1-phénylcyclobutyl)urée,
. N-(2,6-diéthylphényl)-N'-(1-phénylcyclopentyl)urée,
. N-(2,6-diéthylphényl)-N'-[(1-phénylcyclopropyl)-méthyl]urée,
. N-(1-phénylcyclopentyl)-N'-(2,4,6-triméthoxyphényl)urée,
. N-(2,6-diméthylphényl)-N'-[1-(2-thiényl)cyclohexyl]urée,
. N-(2,6-diéthylphényl)-N'-[1-(2-thiényl)cyclohexyl]urée,
. N-[2,6-bis(1-méthyléthyl)]-N'-[1-(2-thiényl)cyclohexyl]urée,
. N-(2,6-diéthylphényl)-N'-[(1-phénylcyclohexyl)méthyl]urée,
. N-(2,6-diméthylphényl)-N'-[(1-phénylcyclopentyl)méthyl]urée,
. N-(2,6-diméthylphényl)-N'-[(1-phénylcyclohexyl)méthyl]urée,
. N-(2,6-diéthylphényl)-N'-[(1-phénylcyclopentyl)méthyl]urée,
. N-[2,6-bis(1-méthyléthyl)phényl]-N'-[(1-phénylcyclopentyl)méthyl]urée,
. N-[2,6-bis(1-méthyléthyl)phényl]-N'-[(1-phénylcyclohexyl)méthyl]urée,
. N-[2-méthyl-6-(1-méthyléthyl)phényl]-N'-[1-(2-thiényl)cyclohexyl]urée,
. N-[2-(1,1-diméthyléthyl)-6-méthylphényl]-N'-[1-(2-thiényl)cyclohexyl]urée,
. N-[2-méthyl-6-(1-méthyléthyl)phényl]-N'-[(1-phénylcyclohexyl)méthyl]urée,
. N-[2-(1,1-diméthyléthyl)-6-méthylphényl]-N'-[(1-phénylcyclohexyl)méthyl]urée,
. N-[2-éthyl-6-(1-méthyléthyl)phényl]-N'-[(1-phénylcyclohexyl)méthyl]urée,
. N-[2-méthyl-6-(1-méthyléthyl)phényl]-N'-[(1-phénylcyclopentyl)méthyl]urée,
. N-[2-(1,1-diméthyléthyl)-6-méthylphényl]-N'-[(1-phénylcyclopentyl)méthyl]urée,
. N-[2-éthyl-6-(1-méthyléthyl)phényl]-N'-[(1-phénylcyclopentyl)méthyl]urée,
. N-(2,4-difluorophényl)-N'-[1-(2-thiényl)-cyclohexyl]-urée,
. N-(2,4-difluorophényl)-N'-[(1-phénylcyclopentyl)méthyl]urée,
. N-(2,4-difluorophényl)-N'-[(1-phénylcyclohexyl)méthyl]urée,
. N-(2,6-dibromo-4-fluorophényl)-N'-[(1-phénylcyclohexyl)méthyl]urée.

**5.** Une composition pharmaceutique destinée à réguler le cholestérol, comprenant une quantité efficace à effet inhibiteur de l'ACAT d'un composé selon les revendications 1 à 4 en combinaison avec un excipient pharmaceutiquement acceptable.

**6.** Utilisation d'un composé selon les revendications 1 à 4 pour la préparation de produits pharmaceutiques destinés à l'inhibition de l'absorption intestinale du cholestérol ou à la régulation du cholestérol.

**7.** Un procédé de préparation de produits pharmaceutiques, par formulation d'un principe actif selon les revendications 1 à 4 avec un excipient inerte pharmaceutiquement acceptable.

**8.** Un procédé de préparation d'un composé selon les revendications 1 à 4, qui comprend les étapes consistant:

a) à faire réagir un composé de formule:

Ar-N = C = O

dans laquelle Ar est tel que défini ci-dessus, avec un composé ayant la formule:

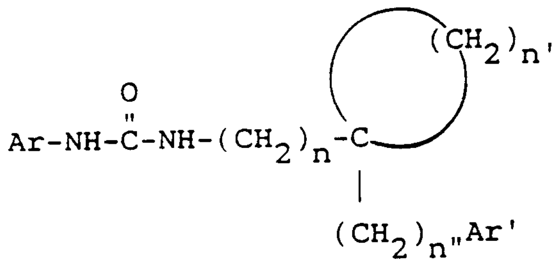

dans laquelle n, n', n" et Ar' sont tels que définis ci-dessus,
dans un solvant organique aprotique polaire à la température ambiante;

b) puis à isoler le produit de ladite étape de réaction; et

c) si on le souhaite, à convertir ledit produit isolé en un sel pharmaceutiquement acceptable.